# EUROPÄISCHE PATENTANMELDUNG

(11) **EP 3 251 626 A2**
(43) Veröffentlichungstag der Anmeldung: **06.12.2017**
(21) Anmeldenummer: 17179691.5
(22) Anmeldetag: 02.02.2012
(51) Int. Cl.: A61C 1/08, A61B 17/17, A61C 13/00

(54) **VERFAHREN ZUR BEREITSTELLUNG EINER LAGEBEZIEHUNG UND ZUR ERSTELLUNG EINER BOHRSCHABLONE**

(30) Priorität: 03.02.2011 DE 102011003561
(62) Teilanmeldung aus: 12706212.3
(71) Anmelder: Sirona Dental Systems GmbH, 64625 Bensheim (DE)
(72) Erfinder: SCHNEIDER, Sascha, 64367 Mühltal (DE)
(74) Vertreter: Sommer, Peter

(57) **Zusammenfassung**

Die Erfindung betrifft Verfahren zur Korrelation einer Implantationsstelle (2) eines Kiefers (1) und 3D-Messdatensatzes (21) der Implantationsstelle (2) mittels eines am Kiefer (1) im Bereich der Implantationsstelle (2) positionierten Abdrucks (3) mit einem Anschlussteil (7) für ein weiteres Bauteil, wobei die Position des Anschlussteils (7) relativ zur Implantationsstelle (2) festgestellt wird. Die Erfindung betrifft weiterhin ein Verfahren zur Erstellung einer Bohrschablone (11) aus einem Abdruck (3) mit einem Anschlussteil (7) und einer an dem Anschlussteil (7) anbringbaren Bohrhilfe (8).

## Beschreibung

### Technisches Gebiet

Die Erfindung betrifft ein Verfahren zur Bereitstellung einer Lagebeziehung zwischen einer Implantationsstelle eines Kiefers und einem 3D-Messdatensatz der Implantationstelle und ein Verfahren zur Erstellung einer Bohrschablone.

### Stand der Technik

Aus dem Stand der Technik sind mehrere Bohrschablonen bekannt, die das kontrollierte Durchführen von geplanten Implantatbohrungen ermöglichen. Durch solche Bohrschablonen kann sichergestellt werden, dass eine erzielte Bohrung eine geplante Bohrrichtung, eine geplante Bohrtiefe oder auch einen geplanten Bohrdurchmesser aufweist.

Die DE 199 52 962 A1 offenbart ein Verfahren zur Erstellung einer Bohrhilfe für ein Zahnimplantat, wobei zunächst eine Röntgenaufnahme des Kiefers und danach eine dreidimensionale optische Vermessung der sichtbaren Oberfläche des Kiefers und der Zähne durchgeführt werden. Die Messdatensätze von der Röntgenaufnahme und der dreidimensionalen optischen Aufnahme werden miteinander korreliert. Anhand der vorhandenen Informationen, wie der Art und der Position des Implantats relativ zu den Nachbarzähnen, wird eine Schablone geplant und generiert, die auf den Nachbarzähnen aufliegt und dadurch die exakte Bohrung des Implantatführungslochs ermöglicht wird. Anhand der Röntgendaten kann das Implantat in bekannter Weise bestimmt und positioniert werden. Anhand der gewonnenen Informationen über die Oberflächenstruktur, d.h. die Okklusalflächen benachbarter Zähne kann eine Implantatshilfe in Gestalt einer Bohrschablone mittels einer CAD/CAM-Einheit ausgeschliffen werden. Anhand der Messdaten ist eine CAD/CAM-Maschine in der Lage die Bohrschablone mit dem Negativ der Okklusalflächen und einem Führungslauf für den Bohrer zu fertigen. Auf die Bohrschablone wird ein Anschlag übertragen, der die Bohrtiefe bestimmt.

Aus der WO 99/32045 ist ein Verfahren zur Herstellung einer dentalen Bohrhilfe für Implantate bekannt, wobei unter Verwendung eines Kieferbildes mit Bezug auf eine Abdruckfläche ein dreidimensionales Rechnerbild modelliert, die Lage und die Bohrtiefe der Bohrlöcher bestimmt und ein Satz von Implantatbohrlochkoordinaten in eine computergesteuerte Fertigungsmaschine eingespeist wird. Mittels eines Präzisionswerkzeugs wird im Bohrkörper für jeden der zuvor eingespeisten Bohrlochkoordinatensätze ein Bohrführungssockel mit einer entsprechend der anhand des Kieferabschnitts ermittelten Bohrlochposition und Bohrlochorientierung vorbereitet.

Ein Nachteil dieser Verfahren besteht darin, dass die meisten CAD/CAM-Maschinen in ihren Freiheitsgraden eingeschränkt sind und daher die Herstellung von Bohrschablonen gemäß der bekannten Verfahren und mittels einer solchen CAD/CAM-Maschine nur für begrenzte Indikationsbereiche möglich ist. Aus diesem Grund erfolgt die Fertigung der Bohrschablone in den meisten Fällen entweder individuell im Labor oder nach vorheriger CAD/CAM-Planung zentral beispielsweise unter Verwendung eines Hexapods, dessen Konstruktion eine Beweglichkeit des zu bearbeitenden Objekts in allen sechs Freiheitsgraden ermöglicht, und mittels eines Parallelometers zur Durchführung senkrechter Bohrungen. Bei der zentralen Fertigung finden meist CT-DVT Schablonen, Aufbissplatten und Kiefermodelle aus Gips Anwendung. Diese werden auf den aufwendig gestalteten Hexapod montiert und mit Hilfe mehrere Messpunkte eingestellt. Dieser Vorgang ist sehr aufwendig und damit auch fehleranfällig.

Die Aufgabe dieser Erfindung besteht darin, ein Verfahren bereitzustellen, das die Herstellung einer beliebigen Bohrschablone auf möglichst einfache und schnelle Weise unter Verwendung einer herkömmlichen Fertigungsmaschine mit eingeschränkten Freiheitsgraden ermöglicht.

### Darstellung der Erfindung

Im folgenden Beschrieben wird ein Abdruck, der an einer Unterseite eine Negativform zumindest eines Teilbereichs eines Kiefers und zumindest einer in diesem Teilbereich des Kiefers liegenden Implantationsstelle umfasst. Der Abdruck kann zumindest teilweise aus einem röntgenstrahlendurchlässigen Material und/oder einem Material besteht, das in einer MRT-Aufnahme möglichst wenig sichtbar ist. Der Abdruck weist im Bereich der Negativform der Implantationsstelle auf einer der Unterseite gegenüberliegenden Oberseite des Abdrucks ein Anschlussteil auf, das gegenüber der Oberseite des Abdrucks als eine Erhebung und/oder eine Vertiefung ausgebildet ist. Diese Erhebung und/oder Vertiefung weist in Richtung ihres Erhöhens oder Absenkens gegenüber der Oberseite des Abdrucks eine Längsachse auf. Die Erhebung und/oder Vertiefung weist eine Geometrie auf, die bezüglich einer Drehung der Geometrie um die Längsachse eindeutig ist.

Ein zahnmedizinischer Abdruck ist eine negative Abformung eines Kiefers oder eines Teils eines Kiefers, also ein Gesamtkieferabdruck oder ein Teilabdruck. Der Abdruck weist somit eine Unterseite mit der Negativform der Kiefers oder eines Teils des Kiefers und eine der Unterseite gegenüberliegende Oberseite auf. Es wird ein anfangs weiches, formbares, später aushärtendes Material, beispielsweise als Ab-Das Anschlussteil des Abdrucks ermöglicht ein einfaches und präzises Anordnen eines weiteren Bauteils an dem Abdruck, beispielsweise durch das Ein- oder Aufsetzten, wozu das weitere Bauteil ebenfalls ein Anschlussteil aufweist. Das Anschlussteil des separaten Bauteils ist ein Bereich oder Teil des Bauteils, der sich zum Anbringen bzw. zum Verbinden des separaten Bauteils mit dem Abdruck eignet. Hierzu weist das Anschlussteil des Bauteils beispielsweise die Negativform des Anschlussteils des Abdrucks auf, so dass es Formschlüssig mit diesem verbunden werden kann. Es ist aber auch möglich, dass das Anschlussteil des separaten Bauteils nur teilweise der Negativform des Anschlussteils des Abdrucks entspricht, wobei lediglich ein partieller Formschluss erreicht wird.

Vorteilhafterweise kann das Bauteil als ein einteiliges oder als ein mehrteiliges Hilfselement ausgebildet sein, das zumindest teilweise aus einem röntgenopaken und/oder aus einem in einer MRT-Aufnahme erkennbaren Material besteht.

Ein Hilfselement ist ein Bauteil, welches im Gegensatz zum Abdruck zumindest teilweise in einer Röntgenaufnahme oder einer MRT-Aufnahme erkennbar ist. Für Röntgenaufnahmen besteht das Hilfselement zumindest teilweise aus röntgenopakem Material. Hinsichtlich MRT-Aufnahmen ist ein Material geeignet, das beispielsweise eine deutlich von der Dichte des Kieferknochens, der Zähne und des umgebenden Weichgewebes zu unterscheidende Dichte bzw. deutlich unterschiedliche Spin-Gitter-Relaxationszeiten aufweist. Es sind beispielsweise leitende Materialien, wie bestimmte Metalle, geeignet.

Vorteilhafterweise weist die Erhebung oder Vertiefung des Anschlussteils mindestens eine an die Oberseite des Abdruckmasse bezeichnet, verwendet, welches im formbaren Zustand auf den Kiefer bzw. den Teil des Kiefers aufgebracht wird, so dass sich die Form des Kiefers bzw. der am Kiefer befindlichen Zähne als Negativ in dem formbaren Material abbilden. In dieser Position wird das aufgebrachte Material ausgehärtet. Im Ausgehärteten Zustand weist der Abdruck eine vollständig feste, nicht verformbare Form oder zumindest eine bei Verformung einer Ausgangsform immer wieder in die Ausgangsform zurückschwingende Form auf.

Der beschriebene Abdruck zeichnet sich dadurch aus, dass er beispielsweise aus einem röntgenstrahlendurchlässigen Material ausgebildet ist. Durch die Röntgenstrahlendurchlässigkeit ist ein beschriebener Abdruck in einer Röntgenaufnahme nicht erkennbar. Dadurch ist es möglich, eine Röntgenaufnahme eines Kiefers eines Patienten mit einem am Kiefer positionierten beschriebener Abdruck zu erzeugen, ohne dass Teile des Kiefers und/oder noch vorhandener Zähne vom Abdruck auf der Röntgenaufnahme verdeckt werden.

Um einen entsprechenden Effekt für MRT-Aufnahmen zu erhalten, ist es möglich ein Isolatormaterial zu verwenden, beispielsweise Polyurethane, Polyethylen, Polymere oder Glasfasern, welches in einer MRT-Aufnahme in der Regel unsichtbar oder wenig sichtbar ist. Geeignet sind alle Materialien, die in einer MRT-Aufnahme nur möglichst wenige Signale erzeugt und insbesondere nicht die von Knochen oder Zähnen erzeugten Signale überdeckt. Ein solches Material sollte insbesondere eine deutlich geringere Dichte als die Dichte des umgebenden Gewebes, wie des Kieferknochens, der Zähne und des umgebenden Weichgewebes aufweisen.

Der beschriebene Abdruck erstreckt sich über eine Implantationsstelle und zumindest einen die Implantationsstelle umgebenden Bereich, z.B. benachbarte Zähne, so dass beispielsweise anhand der benachbarten Zähne ein einfaches eindeutiges wieder Einsetzen des aus dem Kiefer entnommenen Abdrucks möglich ist.

Anschlussteil bezeichnet einen Bereich des Abdrucks, der sich durch eine Geometrie auszeichnet, die es ermöglicht, ein weiteres Bauteil an der Oberseite des Abdrucks anzubringen. Hierbei kann es sich beispielsweise um eine Vertiefung handeln, in die ein weiteres Bauteil zumindest teilweise einsetzbar ist. Das Anschlussteil kann auch als Erhöhung ausgebildet sein, auf die ein weiteres Bauteil aufsetzbar ist.

Die Ausformung des Anschlussteils ist dadurch gekennzeichnet, dass es eine Längsachse aufweist, die in Richtung des Erhöhens oder Absenkens der Anschlussteils relativ zur Oberfläche des Abdrucks verläuft und dass die Außenkontur des Anschlussteils in einer zu dieser Längsachse senkrechten Ebene asymmetrisch ist. Eine solche Asymmetrie in Umlaufrichtung um die Längsachse sichert ein hinsichtlich einer Drehung um die Längsachse eindeutiges Anbringen eines weiteren Bauteils mit einer zum Anbringen geeigneten Geometrie.

Vorteilhafterweise kann der Abdruck mit dem Anschlussteil einteilig ausgebildet werden. Dies kann eine besonders einfach Herstellungsvariante sein, einen beschriebenen Abdruck bereitzustellen.

Vorteilhafterweise kann ein separates Bauteil vorhanden sein, welches ein zumindest teilweise einem Negativ des Anschlussteils des Abdrucks entsprechendes Anschlussteil umfasst und welches zumindest teilweise in das Anschlussteil des Abdrucks einbringbar bzw. auf das Anschlussteil aufsetzbar ist. drucks angrenzende Seitenwand auf. Solche Seitenwände sind insbesondere dazu geeignet das weitere Bauteil durch Formschluss zu halten.

Vorteilhafterweise weist die Vertiefung des Anschlussteils einen gegenüber der Oberseite des Abdrucks abgesenkten Boden oder die Erhebung des Anschlussteils ein gegenüber der Oberseite des Abdrucks erhöhtes Dach auf. Mittels des Bodens bzw. des Dachs des Anschlussteils des Abdrucks kann als Tiefenstop die Position eines anzubringenden Bauteils in Richtung der Erhebung bzw. Vertiefung festgelegt werden. Dies kann beispielsweise dadurch geschehen, dass das anzubringende Bauteil bis zu einem Kontakt einer hierfür geeigneten Fläche des Bauteils mit dem Boden bzw. dem Dach in das Anschlussteil des Abdrucks eingeschoben bzw. auf das Anschlussteil des Abdrucks aufgesetzt wird.

Weiterhin wird eine Bohrschablone für eine an einer Implantationsstelle durchzuführende Bohrung beschrieben, die aus einem vorbeschriebenen Abdruck und mindestens einer Bohrhilfe zur Führung eines Bohrers besteht. Die Bohrhilfe weist ein Anschlussteil zur Verbindung mit dem Abdruck auf, welches zumindest teilweise einem Negativ des Anschlussteils des Abdrucks entspricht. Die Bohrhilfe weist weiterhin eine Durchgangsöffnung auf, die eine Bohrrichtung relativ zu dem Anschlussteil festlegt.

Durch die jeweiligen Anschlussteile des Abdrucks und der Bohrhilfe kann ein Zusammenfügen von Bohrhilfe und Abdruck in einer eindeutigen Position auf einfache Art und Weise sichergestellt werden.

Dadurch kann weiterhin sichergestellt werden, dass die Durchgangsöffnung der Bohrhilfe relativ zum Abdruck und damit bei dem am Kiefer angeordneten Abdruck auch relativ zum Kiefer in einer vorgegebenen Richtung, nämlich einer gewünschten Bohrrichtung, verläuft.

Vorteilhafterweise ist zumindest das Anschlussteil der Bohrhilfe durch Fräsen oder Schleifen herstellbar. Dies ist eine besonders einfache Art der Herstellung. Übliche Fräs- und Schleifmaschinen verfügen allerdings nur über eine begrenzte Anzahl von Freiheitsgraden hinsichtlich der Bearbeitungsrichtungen. Die beschriebene Bohrhilfe ist daher so ausgebildet, dass sie auch mit einer üblichen Fräs- oder Schleifmaschine mit eingeschränkten Freiheitsgraden herstellbar ist.

Vorteilhafterweise weist die Bohrhilfe eine Auflagefläche zur Auflage auf der Oberseite des Abdrucks auf. Dies ist eine weitere Möglichkeit die Position der am Abdruck angeordneten Bohrhilfe zu kontrollieren. Eine solche Auflagefläche kann beispielsweise für eine in ein als Vertiefung ausgebildetes Anschlussteil eines Abdrucks einschiebbare Bohrhilfe oder auch für eine auf ein als Erhöhung ausgebildetes Anschlussteil eines Abdrucks aufsetzbare Bohrhilfe einen Tiefenstop darstellen und damit die Position zumindest hinsichtlich einer in Richtung der Erhöhung bzw. Vertiefung verlaufenden Richtung festlegen.

Vorteilhafterweise ist die Durchgangsöffnung der Bohrhilfe zylindrisch ausgebildet und weist einen Durchmesser auf, der einem festgelegten Bohrdurchmesser entspricht.

Hierdurch kann das Durchgangsloch der Bohrhilfe insbesondere im an den Abdruck angebrachten Zustand zur Führung eines Bohrers dienen.

Vorteilhafterweise umfasst die Bohrhilfe mindestens einen Adapter, wobei der Adapter eine zylindrische Durchgangsöffnung mit einem Durchmesser, der einem festgelegten Bohrdurchmesser entspricht, und eine Außengeometrie mit einem in die Durchgangsöffnung der Bohrhilfe zumindest teilweise formschlüssig hinein passenden Bereich und einem sich an diesen Bereich anschließenden, nicht in die Durchgangsöffnung hinein passenden Abschlussbereich aufweist.

Hierdurch kann die Durchgangsöffnung des Adapters zur Führung eines Bohrers dienen, wobei der Adapter teilweise in die Durchgangsöffnung der Bohrhilfe einbringbar ist, so dass die den Adapter umfassende Bohrhilfe in Verbindung mit dem Abdruck eine Bohrschablonen zur Führung eines Bohrwerkzeugs bildet.

Vorteilhafterweise kann die Durchgangsöffnung der Bohrhilfe oder des Adapters der Bohrhilfe für eine Implantat-Bohrung oder eine Pilot-Bohrung ausgebildet sein.

Der Durchmesser der Durchgangsöffnung der Bohrhilfe selbst oder im Falle einer einen Adapter umfassenden Bohrhilfe der Durchgangsöffnung des Adapter richtet sich nach dem Durchmesser des zu verwendenden Bohrers, wie dies aus dem Stand der Technik für Bohrhilfen im Allgemeinen bereits bekannt ist. Häufig wird mindestens eine erste als Pilot-Bohrung bezeichnete Bohrung mit einem möglichst kleinen Bohrdurchmesser vorgenommen, ehe die Implantat-Bohrung, also eine Bohrung mit einem dem einzusetzenden Implantat entsprechenden Bohrdurchmesser durchgeführt wird. Dies wird durch das Vorsehen mehrerer Bohrhilfen, die jeweils an dem im Bereich der Implantationsstelle am Kiefer angeordneten Abdruck anbringbar sind, möglich. Es ist auch möglich, mehrere Adapter mit Durchgangsöffnungen mit jeweils verschiedenen Durchmessern vorzusehen, die jeweils an der am Abdruck angeordneten Bohrhilfe anbringbar sind.

Vorteilhafterweise kann an einem dem Anschlussteil abgewandten Ende der Bohrhilfe ein Anschlag für ein Bohrwerkzeug bereitgestellt werden, wobei durch die Länge der Durchgangsöffnung die Bohrtiefe vorgebbar ist.

Mit einem Anschlag wird hier eine definierte Fläche oder Kante der Bohrhilfe bezeichnet, die einen Tiefenstop bewirkt, also ein weiteres Eindringen eines Bohrers bzw. Bohreinsatzes des verwendeten Bohrwerkzeugs in die Bohrhilfe verhindert. Hierdurch kann eine Bohrtiefe festgelegt werden.

Vorteilhafterweise kann eine Längsachse der Durchgangsöffnung der Bohrhilfe mit der Längsachse des Anschlussteils der Bohrhilfe einen Winkel α einschließen, der vorzugsweise größer als 0° und kleiner als oder gleich 60° ist. Hierdurch kann sichergestellt werden, dass die Durchgangsöffnung in Richtung einer gewünschten Bohrrichtung verläuft.

Die Erfindung betrifft weiterhin ein Verfahren zur Korrelation einer Implantationsstelle eines Kiefers und eines 3D-Messdatensatzes der Implantationsstelle. Das Verfahren sieht vor einen an der Unterseite eine Negativform zumindest eines Teilbereichs eines Kiefers und zumindest einer in diesem Teilbereich des Kiefers liegenden Implantationsstelle aufweisenden Abdruck aus einem röntgenstrahlendurchlässigen Material und/oder aus Isolatormaterial mit mindestens einem zumindest teilweise röntgenopaken und/oder teilweise in MRT-Aufnahmen sichtbarem Hilfselement bereitzustellen. Das Hilfselement weist ein Anschlussteil auf, das im Bereich der Negativform der Implantationsstelle auf einer der Unterseite gegenüberliegenden Oberseite des Abdrucks am Abdruck angebracht ist. Es wird mindestens eine Röntgenaufnahme und/oder MRT-Aufnahme zumindest des Teilbereichs des Kiefers und der zumindest einen in diesem Teilbereich liegenden Implantationsstelle, des an der Implantationsstelle eingesetzten Abdrucks und des mindestens einen an dem Abdruck angebrachten Hilfselements erstellt und ein 3D-Messdatensatz aus der mindestens einen Röntgenaufnahme und/oder der mindestens einen MRT-Aufnahme erzeugt. Die Position des Anschlussteils des mindestens einen Hilfselements relativ zur Implantationsstelle in dem 3D-Messdatensatz wird festgestellt. Durch Entfernen des Hilfselements wird ein dem Negativ des Anschlussteils des mindestens einen Hilfselements entsprechendes Anschlussteil an einer mit der im Messdatensatz festgestellten Position des Anschlussteils des Hilfselements übereinstimmenden Position in dem an der Implantationsstelle positionierbaren Abdruck bereitgestellt.

Der Zusammenhang zwischen der zu versorgenden Implantationsstelle im Mund eines Patienten und einem 3D-Datensatz wird mittels eines Anschlussteils zum Anschluss weiterer Bautaile bereitgestellt, das im Mund an einem am Kiefer positionierbaren Abdruck angeordnet ist und dessen Position im 3D-Datensatz bekannt ist.

Hierfür wird ein Hilfselement an einem an der Implantationsstelle angeordneten Abdruck angebracht und in einer Röntgenaufnahme oder einer MRT-Aufnahme vermessen. Das Hilfselement zeichnet sich dadurch aus, dass es zumindest teilweise röntgenopak und damit in der Röntgenaufnahme erkennbar ist und/oder in einer MRT-Aufnahme erkennbar ist, während der Abdruck aus röntgenstrahlendurchlässigem und/oder isolierendem Material in der Röntgenaufnahme und/oder der MRT-Aufnahme nicht zu sehen ist. Weiterhin ist in der Röntgenaufnahme bzw. der MRT-Aufnahme die Implantationsstelle erkennbar, so dass aus den Daten der Röntgenaufnahme bzw. der MRT-Aufnahme die relative Position der röntgenopaken bzw. in der MRT-Aufnahme sichtbaren Bereiche des Hilfselements zur Implantationsstelle bestimmt werden kann.

Das Hilfselement weist ein Anschlussteil auf, womit ein Bereich bezeichnet wird, der sich durch eine Geometrie auszeichnet, die es ermöglicht, das Hilfselement an der Oberseite des Abdrucks anzubringen. Hierbei kann es sich beispielsweise um eine Erhöhung handeln, die so an dem Abdruck anbringbar ist, dass sie zumindest teilweise vom Abdruck eingeschlossen wird. Hierzu kann das als Erhöhung ausgebildetes Anschlussteil des Hilfselements beispielsweise in eine noch nicht ausgehärtete Abdruckmasse, die zur Erzeugung des Abdrucks an der Implantationsstelle angebracht wurde, hineingedrückt werden und während des Aushärtens der Abdruckmasse dort verbleiben. Das Anschlussteil wird hierfür möglichst im Bereich der Implantationsstelle auf der die Oberseite des Abdruck ergebenden Seite der Abdruckmasse positioniert.

Es wäre auch möglich ein als Erhöhung ausgebildetes Hilfselement auf der Oberseite des Abdrucks zu positionieren und so mit Abdruckmasse zu umkleiden, dass es nach dem Aushärten dieser Abdruckmasse am Abdruck verbleibt.

Das Anschlussteil des Hilfselements kann aber beispielsweise auch als Vertiefung ausgebildet sein. Eine solche Vertiefung kann beispielsweise durch Aufsetzen auf eine geeignete Erhöhung am Abdruck mit dem Abdruck verbunden werden. Hierfür kann beispielsweise zusätzliche Abdruckmasse auf die Oberseite des Abdrucks aufgebracht werden und das Anschlussteil des Hilfselements auf diese zusätzliche Abdruckmasse so aufgeprägt werden, dass die zusätzliche Abdruckmasse die Vertiefung des Anschlussteils des Hilfselements zumindest teilweise ausfüllt.

Das Hilfselement wird nach der Durchführung der Röntgenaufnahme von dem Abdruck entfernt, so dass am Abdruck zumindest teilweise eine Negativform des Anschlussteils des Hilfselements verbleibt. Dieser Bereich wird wiederum als Anschlussteil des Abdrucks bezeichnet, da er sich dazu eignet ein weiteres Bauteil, welches ein dem Hilfselement entsprechendes Anschlussteil aufweist mit dem Abdruck zu verbinden.

Hat das Hilfselement ein als Erhebung ausgebildetes Anschlussteil, so ist das Anschlussteil des Abdrucks als eine zumindest teilweise dem Negativ dieser Erhebung entsprechende Vertiefung ausgebildet.

Ein weiterer Gegenstand der Erfindung ist ein Verfahren zur Korrelation einer Implantationsstelle eines Kiefers und eines 3D-Messdatensatzes der Implantationsstelle, wobei ein an der Unterseite eine Negativform zumindest eines Teilbereichs eines Kiefers und zumindest einer in diesem Teilbereich des Kiefers liegenden Implantationsstelle aufweisender Abdruck aus einem teilweise röntgenstrahlendurchlässigen Material und/oder aus Isolatormaterial mit mindestens einem röntgenopaken und/oder in MRT-Aufnahmen sichtbaren Bereich bereitgestellt wird, wobei der Abdruck im Bereich der Negativform der Implantationsstelle auf einer der Unterseite gegenüberliegenden Oberseite des Abdrucks ein Anschlussteil aufweist und die Lagebeziehung zwischen dem röntgenopaken und/oder in MRT-Aufnahmen sichtbaren Bereich und dem Anschlussteil bekannt ist. Weiterhin wird mindestens eine Röntgenaufnahme und/oder MRT-Aufnahme zumindest des Teilbereichs des Kiefers, der zumindest einen in diesem Teilbereich liegenden Implantationsstelle und des an der Implantationsstelle eingesetzten Abdrucks hergestellt und ein 3D-Messdatensatz aus der mindestens einen Röntgenaufnahme und/oder der mindestens einen MRT-Aufnahme erzeugt. Die Position des röntgenopaken und/oder in der MRT-Aufnahme sichtbaren Bereichs des Abdrucks relativ zur Implantationsstelle in dem 3D-Messdatensatz wird festgestellt und die relative Position des Anschlussteils des Abdrucks zur Implantationsstelle aufgrund der relativen Position des röntgenopaken und/oder in der MRT-Aufnahme sichbaren Bereichs bestimmt.

Wie das bereits beschriebene Verfahren stellt dieses erfindungsgemäße Verfahren einen Zusammenhang zwischen einer Implantationsstelle im Mund eines Patienten und einem 3D-Datensatz her. Das am Abdruck angeordnete Anschlussteil, dessen Lage im 3D-Datensatz bekannt ist, kann als Orientierungshilfe zur Übertragung von Positionen und Richtungen aus dem 3D-Datensatz in den realen Raum im Mund des Patienten dienen.

Hierfür wird bei diesem weiteren erfindungsgemäßen Verfahren eine Röntgenaufnahme und/oder eine MRT-Aufnahme eines an der Implantationsstelle angeordneten Abdrucks erzeugt. Der Abdruck weist ein Anschlussteil, also einen Bereich auf, der dazu geeignet ist, ein weiteres Bauteil am Abdruck anzubringen. Weiterhin besteht der Abdruck zu möglichst großen Teilen aus einem röntgenstrahlendurchlässigen Material, das in der Röntgenaufnahme nicht erkennbar ist, und/oder aus einem Isolatormatieral, das in der MRT-Aufnahme nicht sichtbar ist. Nur ein kleinerer Bereich sollte aus einem röntgenopakten und daher im Röntgenbild sichtbaren Material und/oder aus einem in der MRT-Aufnahme erkennbaren Material, beispielsweise einem leitenden Material, bestehen. Die Lage dieses röntgenopaken und/oder in der MRT-Aufnahme erkennbaren Bereichs des Abdrucks zum Anschlussteil ist bekannt. Dadurch werden einerseits möglichst wenige Bereiche des Kiefers und der Implantationsstelle in der Röntgenaufnahme bzw. der MRT-Aufnahme durch den Abdruck verdeckt, andererseits kann die relative Position des Anschlussteils des Abdrucks zur Implantationsstelle ermittelt werden.

Ein weiterer Gegenstand der Erfindung ist ein Verfahren zur Korrelation einer Implantationsstelle eines Kiefers und eines 3D-Messdatensatzes der Implantationsstelle, wobei ein an der Unterseite eine Negativform zumindest eines Teilbereichs eines Kiefers und zumindest einer in diesem Teilbereich des Kiefers liegenden Implantationsstelle aufweisender Abdruck aus einem röntgenstrahlendurchlässigen Material und/oder Isolatormaterial bereitgestellt wird. Weiterhin wird mindestens ein optischen Hilfselement bereitgestellt, welches ein Anschlussteil aufweist, das im Bereich der Negativform der Implantationsstelle am Abdruck von einer der Unterseite gegenüberliegenden Oberseite des Abdrucks den Abdruck durchdringt und über die Unterseite des Abdrucks hinausreicht und an dem über die Unterseite des Abdruck hinausragenden Ende mindestens einen fest oder abnehmbar angeordneten Findekörper aufweist. Es wird mindestens eine Röntgenaufnahme und/oder eine MRT-Aufnahme zumindest des Teilbereichs des Kiefers und der zumindest einen in diesem Teilbereich liegenden Implantationsstelle erstellt und ein 3D-Messdatensatz aus der mindestens einen Röntgenaufnahme und/oder der mindestens einen MRT-Aufnahme erzeugt. Weiterhin wird mindestens eine optische Aufnahme der Unterseite des Abdrucks mit dem eingesetzten und über die Unterseite des Abdrucks hinausragenden optischen Hilfselement mit dem mindestens einen am hinausragenden Anschlussteil des Hilfselements angebrachten Findekörper erstellt und ein weiterer 3D-Messdatensatz aus der mindestens einen optischen Aufnahme erzeugt. Aus den erzeugten 3D-Messdatensätzen wird durch Korrelation ein Korrelationsmessdatensatz erzeugt und die Position des Anschlussteils des mindestens einen optischen Hilfselements relativ zur Implantationsstelle in dem Korrelationsdatensatz bestimmt. Durch Entfernen des optischen Hilfselements wird ein dem Negativ des Anschlussteils des mindestens einen optischen Hilfselements entsprechendes Anschlussteil an einer mit der im Korrelationsdatensatz festgestellten Position des Anschlussteils des optischen Hilfselements übereinstimmenden Position in dem an der Implantationsstelle eingesetzten Abdruck bereitgestellt.

Wie die bereits beschriebenen Verfahren stellt dieses erfindungsgemäße Verfahren einen Zusammenhang zwischen einer Implantationsstelle im Mund eines Patienten und einem Messdatensatz her. Hierfür werden bei diesem weiteren erfindungsgemäßen Verfahren eine Röntgenaufnahme und/oder eine MRT-Aufnahme eines an der Implantationsstelle angeordneten Abdrucks und eine optische Aufnahme einer Unterseite des Abdrucks korreliert.

Die Röntgenaufnahme und/oder MRT-Aufnahme wird von dem Bereich des Kiefers angefertigt, der die Implantationsstelle umfasst und dessen Negativform auf der optisch zu vermessenden Unterseite des Abdrucks eingeprägt ist.

Für die optische Aufnahme ist ein optisches Hilfselement am Abdruck angebrachten, das ein Anschlussteil aufweist, welches von der Oberseite des Abdrucks durch den Abdruck hindurch reicht, so dass es über die Unterseite des Abdrucks hinausragt. An dem über die Unterseite hinausragenden Ende ist während der optischen Aufnahme mindestens ein Findekörper angeordnet.

Ein Findekörper zeichnet sich dadurch aus, dass er eine spezifische äußere Form aufweist, die in einer optischen Aufnahme gut erkennbar ist, so dass die genaue Lage des Findekörpers in der optischen relativ zur aufgenommenen Oberfläche der Unterseite des Abdrucks in der optischen Aufnahme bestimmt werden kann. Aus der bekannten relativen Lage des Findekörpers zum Anschlussteils des optischen Hilfselements kann somit auf die Position des Anschlussteil des optischen Hilfselements in der optischen Aufnahme geschlossen werden, auch wenn diese teilweise im Abdruck liegt und daher auf der optischen Aufnahme selbst nicht erkennbar ist.

Die Erzeugung des Korrelationsdatensatzes kann nach einem aus dem Stand der Technik bekannten Verfahren zur Korrelation von Röntgendaten und optischen Daten, beispielsweise dem in der DE 199 52 962 A1 beschriebenen Verfahren, vorgenommen werden.

Mittels der Korrelation wird ein Zusammenhang zwischen der hinsichtlich der optischen Aufnahme bekannten Position des Anschlussteils des optischen Hilfselements und der Röntgenaufnahme hergestellt. Damit ist es möglich zu allen Punkten bzw. Positionen der Röntgenaufnahme, beispielsweise eine in der Röntgenaufnahme festgelegte Bohrrichtung oder Bohrtiefe einer Implantatbohrung, eine entsprechende relative Position zum Anschlussteil des am Abdruck angeordneten optischen Hilfselements bzw. nach Entfernen des optischen Hilfselements zum Anschlussteil des Abdrucks zu bestimmen.

Ein weiterer Gegenstand der Erfindung ist ein Verfahren zur Erstellung einer Bohrschablone für eine an einer Implantationsstelle durchzuführende Bohrung, wozu ein an der Unterseite eine Negativform zumindest eines Teilbereichs eines Kiefers und zumindest einer in diesem Teilbereich des Kiefers liegenden Implantationsstelle aufweisender Abdruck mit mindestens einem Anschlussteil mit bekannter Anschlussgeometrie bereitgestellt wird, wobei das Anschlussteil im Bereich der Negativform der Implantationsstelle auf einer der Unterseite gegenüberliegenden Oberseite des Abdrucks angeordnet ist und dessen Lagebeziehung zum Kiefer bei an der Implantationsstelle positioniertem Abdruck bekannt ist. Weiterhin wird eine Bohrrichtung für mindestens ein Implantat relativ zu dem Anschlussteil des Abdrucks anhand eines die Implantationsstelle enthaltenden 3D-Messdatensatzes des Kiefers festgelegt und mindestens eine Bohrhilfe mit einem Anschlussteil aus einem Block mit einer entlang einer Längsachse des Blocks verlaufenden Durchgangsöffnung hergestellt, wobei das Anschlussteil zumindest teilweise dem Negativ des Anschlussteils des Abdrucks entspricht und wobei das Anschlussteil in einem Winkel zu der Durchgangsöffnung angeordnet wird, der einem Winkel der Bohrrichtung zu dem Anschlussteil des Abdrucks entspricht. Die mindestens eine Bohrhilfe wird an dem Abdruck angebracht, wobei eine korrekte Orientierung der Bohrhilfe am Abdruck mittels der Anschlussteile sichergestellt wird.

Eine erfindungsgemäße Bohrschablone wird aus einem Abdruck und einer Bohrhilfe erstellt.

Der Abdruck weist hierfür eine Unterseite mit einer Negativform einen Teilbereichs eines Kiefers auf, wobei der Teilbereich zumindest eine Implantationsstelle umfasst. Weiterhin weist der Abdruck eines Anschlussteil auf, die im Bereich der Implantationsstelle an einer der Unterseite gegenüberliegenden Oberseite des Abdrucks angeordnet ist. Das Anschlussteil ist ein Bereich des Abdrucks, der eine bekannte Geometrie aufweist, die dazu geeignet ist ein weiteres Bauteil daran anzubringen. Es kann sich beispielsweise um eine Vertiefung handeln, in die ein weiteres Bauteil zumindest teilweise einsteckbar ist. Das Anschlussteil kann aber auch als Erhöhung ausgebildet sein, auf die ein weiteres Bauteil aufsteckbar ist. Weiterhin ist die Lage des Anschlussteils zum Kiefer bekannt.

In einem 3D-Messdatensatz, der die Implantationsstelle enthält, werden die Parameter der Implantatbohrung insbesondere die Bohrrichtung, aber auch Bohrdurchmesser und/oder Bohrrichtung und Bohrtiefe festgelegt. Mittels der bekannten Lagebeziehung zwischen der Implantationsstelle und dem Anschlussteil des Abdrucks werden diese Größen relativ zum Anschlussteil des Abdrucks bestimmt.

Anhand dieser Parameter wird durch Anordnen eines Anschlussteils an einem Block eine Bohrhilfe hergestellt, wobei der Block bereits eine entlang einer Längsachse verlaufende Öffnung aufweist. Das herzustellende Anschlussteil zeichnet sich dadurch aus, dass es entlang der Längsachse des Blocks an einem Ende des Blocks und hinsichtlich einer Längsachse des Anschlussteils unter einem Winkel zur Längsachse des Blocks angeordnet wird. Das Anschlussteil weist eine Form auf, die zumindest teilweise dem Negativ des Anschlussteils des Abdrucks entspricht, so dass die Bohrhilfe an dem Abdruck durch verbinden der Anschlussteile angebracht werden kann. Diese Verbindung kann durch einen zumindest partiellen Formschluss erreicht werden. Es kann sich beispielsweise um eine Steckverbindung handeln.

Wird der Block während der Herstellung des Anschlussteils an einem Halter gehalten, so wird er von diesem nach Beenden der Herstellung entlang einer zur Öffnung in einem Winkel stehenden Ebene abgetrennt, so dass als Bohrhilfe ein Bauteil mit einer Anschlussgeometrie und einer Durchgangsöffnung bereitgestellt wird.

Der Winkel zwischen dem herzustellenden Anschlussteil der Bohrhilfe und der Öffnung des für die Bohrhilfe verwendeten Blocks wird so gewählt, dass er dem Winkel der festgelegten Bohrrichtung zum Anschlussteil des Abdrucks entspricht. Dadurch verläuft die Durchgangsöffnung der Bohrhilfe nach dem einsetzten der Bohrhilfe in den Abdruck entsprechend der festgelegten Bohrrichtung.

Der richtungsmäßige Verlauf und die Position der Ebene, an der der Block von einem Halter abgetrennt wird bzw. des dem Anschlussteil abgewandten Endes des Blocks kann die Bohrtiefe beispielsweise durch einen Anschlag am zu verwendenden Bohrer vorgeben oder zumindest eine Kontrollmöglichkeit darstellen. Es ist beispielsweise möglich, mittels der nach dem Abtrennen entstandenen Fläche bzw. Kante bzw. mittels der abschließenden Fläche des Blocks einen Tiefenstop bzw. Anschlag für ein Bohrwerkzeug bereitzustellen, welcher ein weiteres Eindringen des Bohrers verhindert und somit eine maximale Bohrtiefe festlegt.

Vorteilhafterweise wird zusätzlich zu der Bohrrichtung ein Bohrdurchmesser für mindestens ein Implantat relativ zum Anschlussteil des Abdrucks festgelegt und ein Block mit einer zylindrischen Öffnung mit einem Durchmesser gemäß einem festgelegten Bohrdurchmesser verwendet.

Der Durchmesser der Öffnung des zu verwendenden Blocks kann so gewählt werden, dass er, wie dies für Bohrschablonen bereits bekannt ist, dem gewünschten Bohrdurchmesser in geeigneter Weise entspricht, um eine sichere Führung eines Bohrers in der Bohrschablone sicherzustellen.

Vorteilhafterweise wird ein Bohrdurchmesser für mindestens ein Implantat relativ zu dem Anschlussteil des Abdrucks festgelegt und ein Block mit einer Öffnung mit einem Durchmesser, der größer als der festgelegte Bohrdurchmesser ist, zur Herstellung der Bohrhilfe verwendet, wobei die Bohrhilfe wird zusammen mit einem Adapter verwendet wird, der eine Bereich mit einer dem Negativ der Öffnung des Blocks entsprechenden Außengeometrie, einem sich diesem Bereich anschließenden Abschlussbereich und einer Durchgangsöffnung mit einem Innendurchmesser gemäß dem festgelegten Bohrdurchmesser aufweist und auf der dem Anschlussteil der Bohrhilfe abgewandten Seite der aus dem Block gefertigten Bohrhilfe in die Durchgangsöffnung der Bohrhilfe zumindest teilweise eingesetzt wird.

Das Herstellen der Bohrhilfe aus einem Block mit einem Durchmesser, der größer als der Bohrdurchmesser ist, ermöglicht es Adapter vorzusehen, die dazu geeignet sind, diesen Durchmesser auf die gewünschte Größe zu reduzieren. Hierzu weist der Adapter eine Durchgangsöffnung mit dem gewünschten Durchmesser, beispielsweise dem Bohrdurchmesser oder einem für eine Pilotbohrung geeigneten Durchmesser, und einer äußeren Form auf, die es ermöglicht den Adapter zumindest teilweise so in die Durchgangsöffnung der Bohrhilfe hinein zu stecken, dass die Durchgangsöffnung des Adapter parallel zur Durchgangsöffnung der Bohrhilfe und teilweise innerhalb der Durchgangsöffnung des Bohrhilfe verläuft. Hierdurch wird eine Bohrschablone aus Abdruck und Bohrhilfe mit Adapter mit einem gewünschten Bohrdurchmesser bereitgestellt. Es ist auch möglich, durch vorsehen mehrerer Adapter mit verschiedenen Durchmessern der Durchgangsöffnung eine Bohrschablone bereitzustellen, die auf einfache weise, nämlich durch austauchen der Adapter für eine erste Pilotbohrung und auch für weitere Bohrungen bzw. die Implantatbohrung verwendbar ist.

Vorteilhafterweise wird zumindest das Anschlussteil der Bohrhilfe aus dem Block gefräst oder geschliffen. Dies ist eine möglichst einfache Variante der Herstellung, die insbesondere durch die Verwendung von Blocks mit vorgebohrter Öffnung und durch eine schräge Anordnung der Anschlussgeometrie ermöglicht wird.

Vorteilhafterweise wird der Block zumindest während der Herstellung des Anschlussteils auf einem Halter gehalten und vor dem Einfügen entlang einer senkrecht oder schräg zur Längsachse des Blocks verlaufenden Abtrennfläche von dem Halter abgetrennt.

Der richtungsmäßige Verlauf und die Position der Ebene, an der der Block von einem Halter abgetrennt wird, an dem er während der Herstellung der Anschlussgeometrie gehalten wird, kann sich nach einer festgelegten Bohrtiefe richten. So ist es möglich, mittels der nach dem Abtrennen entstandenen Fläche bzw. Kante einen Tiefenstop bzw. Anschlag für ein Bohrwerkzeug bereitzustellen, welches ein weiteres eindringen des Bohrers verhindert und somit eine maximale Bohrtiefe festlegt.

### Kurzbeschreibung der Zeichnungen

Ausführungsbeispiele der Erfindung sind in der Zeichnung dargestellt. Es zeigt die
- Fig. 1: einen erfindungsgemäßen Abdruck einer Implantationsstelle und eines Teils eines benachbarten Kiefers mit einem Hilfselement, die
- Fig. 2: das Hilfselement aus Fig. 1, die
- Fig. 3: den Abdruck aus Fig. 1, die
- Fig. 4: eine Bohrhilfe für eine erfindungsgemäße Bohrschablone, die
- Fig. 5: einen Adapter, die
- Fig. 6: schematisch die Herstellung einer Bohrhilfe aus einem Block, die
- Fig. 7: eine weitere Variante eines aus einer aus einem Block herzustellenden Bohrhilfe, die
- Fig. 8: eine erfindungsgemäße Bohrschablone, die
- Fig. 9: ein erfindungsgemäßes optisches Hilfselement, die
- Fig. 10: einen aus einer optischen Vermessung gewonnenen Messdatensatz.

### Ausführungsbeispiele

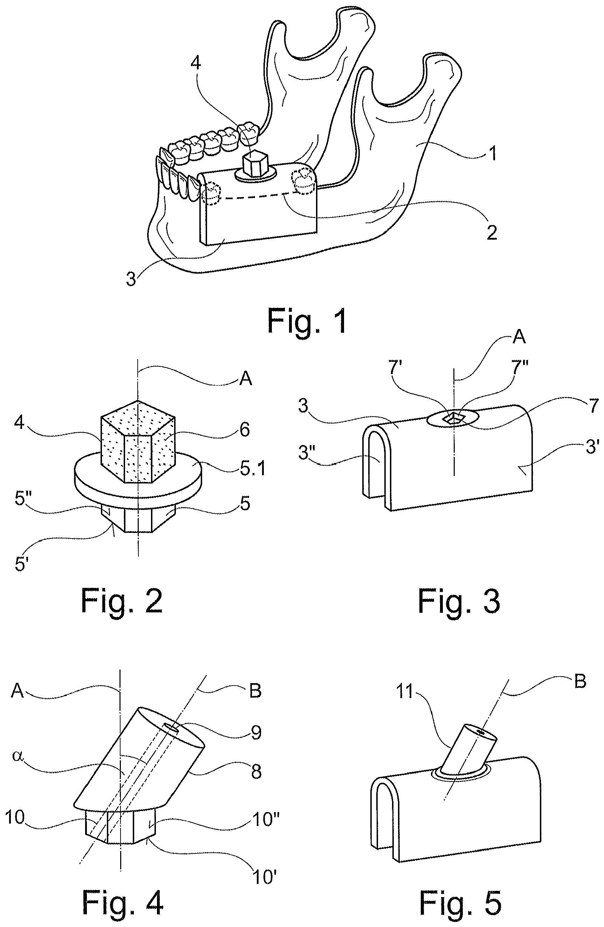

Die Fig. 1 zeigt einen Teil eines Kiefers 1, hier beispielhaft einen Unterkiefer, umfassend eine Implantationsstelle 2, also eine Stelle im Kiefer 1 bzw. ein Bereich des Kiefers 1, an dem ein Implantat im Kiefer 1 angebracht werden soll. Die dargestellte Implantationsstelle 2 befindet sich in einer Schaltlücke am Unterkiefer 1, in deren Bereich mindestens eine Implantat-Bohrung für mindestens ein Implantat angebracht werden soll. Unter Schaltlücke wird hier eine von anderen Zähnen begrenzte Zahnlücke verstanden. Es könnte sich aber auch um eine Implantationsstelle 2 am Oberkiefer 1 oder auch um eine von mehreren Implantationsstellen 2 in einem komplett zahnlosen Kiefer 1 oder um eine Freiendlücke, also eine Zahnlücke, die nur an einer Seite von anderen Zähnen begrenzt ist, handeln.

Aus einer in dem die Implantationsstelle 2 umfassenden Bereich des Kiefers 1 angebrachten und zumindest im verfestigten Zustand röntgenstrahlendurchlässigen und/oder isolierenden Abdruckmasse wird ein Abdruck 3 gebildet. Die Abdruckmasse kann beispielsweise schnell aushärtend sein. Wesentlich ist, dass sich die Abdruckmasse im verfestigter Form, also als Abdruck 3, nicht weiter verformt oder alternativ bei Verformung aus einer Ausgangsform elastisch in die Ausgangsform zurückfindet.

Ein weiteres wesentliches Merkmal ist, dass die der Abdruck in einer Röntgenaufnahme oder in einer MRT-Aufnahme nicht erkennbar ist. Hierfür ist die Abdruckmasse zumindest in verfestigtem Zustand röntgenstrahlendurchlässig oder aus einem isolierenden Material. Isolierende Materialien wie Polyethylene, Polyurethane, Polymere oder Glasfasern sind in der Regel in einem MRT Bild nicht sichtbar.

Der Abdruck 3 weist eine Unterseite 3" mit einer Negativform des die Implantationsstelle 2 umfassenden Bereichs des Kiefers 1 und eine der Unterseite 3" gegenüberliegenden Oberseite 3' auf. Die Nachbarzähne dienen als Orientierung bzw. zur Positionierung des Abdrucks an der Implantationsstelle 2.

Vor dem Aushärten der Abdruckmasse wird im Bereich des geplanten Implantats an der vom Unterkiefer 1 bzw. der Implantationsstelle 2 abgewandten Seite des Abdrucks, also an der Oberseite 3' des Abdrucks 3, ein Hilfselement 4 zumindest teilweise in die Abdruckmasse eingebracht oder an der Abdruckmasse angebracht. Dies kann beispielsweise durch nachträgliches Einpressen in die bereits aufgebrachte, aber noch nicht ausgehärtete Abdruckmasse oder durch ein Aufsetzen und anschließendes Umspritzen oder Umkneten mit weiterer Abdruckmasse geschehen.

Sollen mehrere Implantate angebracht werden, so wird für jedes dieser geplanten Implantate im Bereich der jeweiligen geplanten Position ein Hilfselement 4 zumindest teilweise in die Abdruckmasse eingebracht oder an der Abdruckmasse angebracht. Hinsichtlich jedes einzelnen geplanten Implantats wird so verfahren, wie es hier beispielhaft für ein geplantes Implantat beschrieben wird.

Das Hilfselement 4, welches in Fig. 2 im Detail dargestellt ist, weist mindestens einen röntgenopaken oder in einer MR-T-Aufnahme erkennbaren Bereich 6 sowie ein Anschlussteil 5 zum Anbringen an dem Abdruck 3 auf. Das Hilfselement kann beispielsweise zum Teil aus einem in MRT-Aufnahmen erkennbaren Material bestehen, beispielsweise einem elektrisch leitenden Material wie Metall.

Unter Anschlussteil 5 wird ein Bereich des Hilfselements 4 verstanden, der sich aufgrund seiner Geometrie eignet, dass Hilfselement an dem Abdruck 3 anzubringen. Diese Geometrie des Anschlussteils 5 ist bekannt. Des Weiteren ist auch die Lagebeziehung des Anschlussteils 5 des Hilfselements 4 zu dem röntgenopaken und/oder in einer MRT-Aufnahme sichtbaren Bereich 6 des Hilfselements 4 sowie die Geometrie des röntgenopaken Bereichs selbst bekannt.

Der röntgenopake und/oder in einer MRT-Aufnahme sichtbare Bereich 6 ist ein beliebig ausgestalteter und beliebig angeordneter Bereich des Hilfselements 4, der sich dadurch auszeichnet, dass er röntgenopak, also auf einer Röntgenaufnahme erkennbar ist. Der Bereich 6 kann beispielsweise aus mehreren, z.B. drei Kugeln aus röntgenopakem und/oder in einer MRT-Aufnahme erkennbarem Material bestehen, die nebeneinander auf dem Anschlussteil 5 des Hilfselements 4 oder auf einem sich an das Anschlussteil 5 anschließenden Bereich des Hilfselements 4 angeordnet sind. Der Bereich 6 kann sich auch über das Anschlussteil 5 erstrecken. Es wäre beispielsweise möglich, dass sich der röntgenopake und/oder in einer MRT-Aufnahme sichtbare Bereich 6 über das gesamte Hilfselement 4 erstreckt, dieses also vollständig röntgenopak bzw. in einer MRT-Aufnahme sichtbar ist.

In dem in Fig. 2 dargestellten Ausführungsbeispiel ist das Anschlussteil 5 als eine Erhebung mit einer in Richtung der Erhebung verlaufenden Längsachse A ausgebildet. Die Erhebung weist mehrere parallel zur Längsachse verlaufende Seitenwände 5" auf, die zusammen einen fünfeckigen Querschnitt bilden und durch einen flachen senkrecht zur Längsachse A des Anschlussteils 5 verlaufenden Boden 5' abgeschlossen werden. Die Längsachse A entspricht der Anschlussrichtung des Hilfselements 4, also die Auf- oder Einsteckrichtung, entlang derer das Anschlussteil 5 des Hilfselements 4 in den Abdruck 3 eingebracht wird. Die Seiten des fünfeckigen Querschnitts weisen verschiedene Längen auf, so dass das als Erhebung ausgebildete Anschlussteil 5 bezüglich der Längsachse A eine winkelmäßig eindeutige Geometrie aufweist. Wird das Hilfselement aus dem Abdruck herausgelöst und wieder eingesetzt, so verhindert die winkelmäßig eindeutige Geometrie ein verdrehtes Einsetzen.

Das Anschlussteil 5 des Hilfselements 4 könnte beispielsweise auch als Vertiefung mit einer in Richtung der Absenkung verlaufenden Längsachse ausgebildet sein. Das Anschlussteil 5 könnte auch nur eine einzige Seitenwand 5" aufweisen, die im Querschnitt beispielsweise eiförmig ist. Die Seitenwände können auch in einem Winkel bezüglich der Längsachse des Anschlussteiles 5 verlaufen. Wesentlich ist lediglich, dass die Geometrie des Anschlussteils einen Bereich mit einer bezüglich seiner Längsachse A winkelmäßig eindeutige Geometrie aufweist. So könnte das Anschlussteil beispielsweise als Stempel mit ovalem Querschnitt ausgebildet sein. Der Querschnitt könnte sich beispielsweise auch in Richtung vom übrigen Hilfselement 4 weg verjüngen, wodurch es sich leichter aus dem Abdruck herauslösen lässt.

Das in Fig. 2 dargestellte Hilfselement 4 weist einen röntgenopaken Bereich 6 auf, der als röntgenopake Erhebung mit einer dem Anschlussteil 5 ähnlichen Geometrie ausgebildet und als gepunkteter Bereich dargestellt ist.

Das in Fig. 2 dargestellte Hilfselement 4 weist darüber hinaus einen zwischen dem Anschlussteil 5 und dem röntgenopaken und/oder in einer MRT-Aufnahme sichtbaren Bereich 6 angeordneten tellerförmigen Bereich 5.1 auf. Der tellerförmig, das Anschlussteil abschließende Bereich 5.1 kann beim Einstecken des Hilfselements 4 in den Abdruck 3 als Auflagefläche auf der Oberseite 3' des Abdrucks 3 und damit als eine Art Anschlag für die Einstecktiefe des Hilfselements 4 dienen. Ein erfindungsgemäßes Hilfselement 4 muss einen solchen tellerförmigen Bereich 5.1 nicht aufweisen.

Das Hilfselement 4 kann, wie im dargestellten Fall, einteilig ausgebildet sein. Es ist auch möglich, das Hilfselement 4 mehrteilig auszubilden. Es ist beispielsweise möglich, das Hilfselement 4 aus zwei zusammensteckbaren Bauteilen aufzubauen, wovon das eine Bauteil das Anschlussteil 5 umfasst und das andere Bauteil beispielsweise mindestens einen röntgenopaken und/oder in einer MRT-Aufnahme sichtbaren Bereich 6 aufweist. Hierdurch wäre es beispielsweise möglich zuerst nur den das Anschlussteil 5 umfassenden Teil in der Abdruckmasse des Abdrucks 3 im Mund des Patienten zu positionieren und während des Aushärtens der Abdruckmasse im Mund zu belassen. Dadurch dass dieser erste Teil beispielsweise allein nicht so weit über die Oberseite 3' der Abdruckmasse bzw. des Abdrucks 3 hinausragt, wird ein Verschieben des positionierten Teils des Hilfselements 4 durch die Zunge des Patienten im noch nicht ausgehärteten Zustand der Abdruckmasse verhindert.

Durch Anbringen des Anschlussteils 5 des Hilfselements 4 durch Ein- oder Aufstecken auf der Oberseite 3' des Abdrucks 3 an der noch nicht verfestigte Abdruckmasse und ein anschließendes Entfernen des Hilfselements 4 nach dem Aushärten des Abdrucks 3 bildet sich auf der Oberseite im Abdruck 3 ein Anschlussteil 7 aus, das dem Negativ des Anschlussteils 5 des Hilfselements 4 entspricht.

Im dargestellten Ausführungsbeispiel bildet sich, wie in Fig. 3 dargestellt, ein als Vertiefung bezüglich der Oberseite 3' des Abdrucks ausgebildetes Anschlussteil 7 mit einer in Richtung der Vertiefung verlaufenden Längsachse A, einem Boden 7' und einer bezüglich der Längsachse A winkelmäßig eindeutigen Geometrie auf. Hierdurch wird ein winkelmäßig eindeutiges einsetzen eines in das Anschlussteil 7 des Abdrucks 3 passenden Bauteils gewährleistet.

Es ist auch möglich, dass das Anschlussteil 5 des Hilfselements 4 den Abdruck 3 in Richtung der Längsachse A des sich im Abdruck 3 ausbildenden Anschlussteils 7 vollständig durchbricht, so dass das Anschlussteil 7 des Abdrucks 3 keinen Boden 7' aufweist.

Ist das Anschlussteil 5 des Hilfselements als Vertiefung ausgebildet, so kann eine als Negativ dieser Anschlussgeometrie 5 ausgebildete Anschlussgeometrie 7 an der Oberseite 3' des Abdrucks 3 beispielsweise gebildet werden, indem ein Überschuss an Abdruckmasse auf die Oberseite 3' des Abdrucks 3 aufgebracht und durch aufsetzen der Anschlussgeometrie 5 des Hilfselements 4 geformt wird. Das sich als Erhebung ausbildende Anschlussteil 7 weist dann statt eines Bodens ein Dach auf.

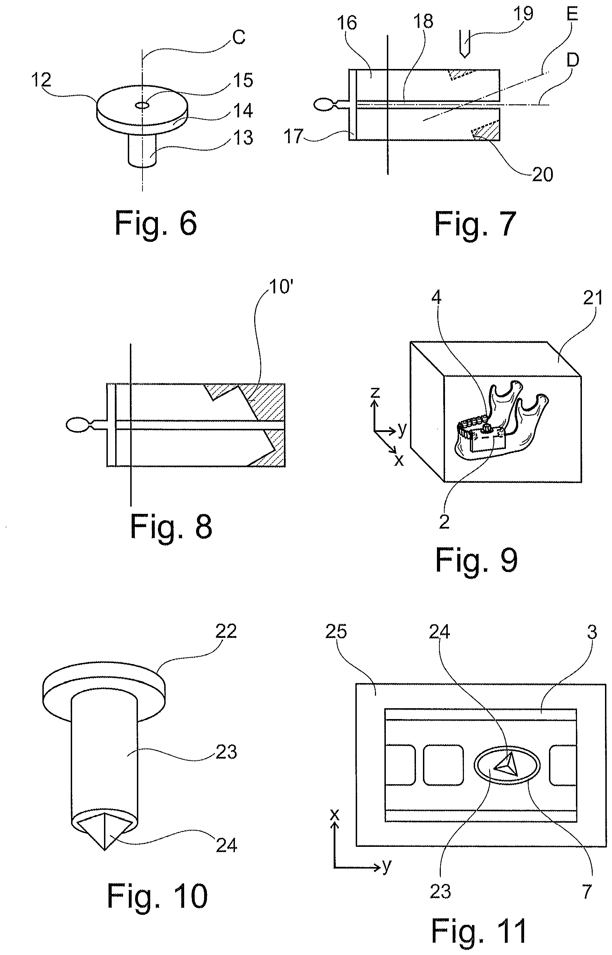

Der an der Implantationsstelle 2 positionierte Abdruck 3 mit dem Anschlussteil 7 mit bekannter Geometrie dient als Orientierungshilfe, indem ein Zusammenhang zwischen der Implantationsstelle 2 am Kiefer 1 und einem 3D-Messdatensatz 21 von der Implantationsstelle 2 hergestellt wird. Hierzu wird mindestens eine Röntgenaufnahme und/oder MRT-Aufnahme der Implantationsstelle 2 mit dem dort positionierten Abdruck 3 und dem daran angebrachten Hilfselement 4 erzeugt. Der Abdruck 3 ist in dem aus der mindestens einen Röntgenaufnahme und/oder MRT-Aufnahme ermittelten mindestens einen 3D-Datensatz 21 nicht erkennbar. Im 3D-Datensatz 21 erkennbar sind sowohl die Implantationsstelle 2 als auch der mindestens eine röntgenopake bzw. in einer MRT-Aufnahme sichtbare Bereich 6 des Hilfselements 4, wie es in Fig.9 schematisch dargestellt ist. Aufgrund der Bekannten Lagebeziehung zwischen dem mindestens einen röntgenopaken und/oder in einer Röntgenaufnahme sichtbaren Bereich 6 des Hilfselements 4 und dem Anschlussteil 5 des Hilfselements 4 wird die relative Position des Anschlussteils 5 des Hilfselements 4 zu der im 3D-Datensatz erkennbaren Implantationsstelle 2 bestimmt. Da die Lage des Anschlussteils 5 des an dem Abdruck 3 angebrachten Hilfselements 4 mit der Lage des Anschlussteils 7 des Abdrucks 3 übereinstimmt, ist damit auch die Lagebeziehung des Anschlussteils 7 des Abdrucks 3 zur Implantationsstelle 2 bekannt. Damit stellt der an der Implantationsstelle 2 positionierte Abdruck 3 mittels seines Anschlussteils 7 nach Entfernen des Hilfselements 4 eine Orientierungshilfe dar, die einen Zusammenhang zwischen der Implantationsstelle 2 am Unterkiefer 1 und dem mindestens einen 3D-Messdatensatz 21 herstellt.

Hierdurch ist es möglich eine Verlaufsrichtung einer geplanten Implantatbohrung, im Folgenden als Bohrrichtung bezeichnet, im 3D-Datensatz 21 festzulegen und anschließend die Lagebeziehung der Bohrrichtung zu dem Anschlussteil 7 des am Unterkiefer 1 positionierten Abdrucks 3 zu bestimmen. Insbesondere kann ein Winkel zwischen der Längsachse A des Anschlussteils 7 des Abdrucks 3 und der Bohrrichtung sowie eine winkelmäßige Ausrichtung der Bohrrichtung bezüglich der Längsachse A des Anschlussteils 7 des Abdrucks 3 bestimmt werden.

Aufgrund dieser Kenntnisse kann eine erfindungsgemäße Bohrhilfe 8 hergestellt werden, wie sie in Fig. 4 dargestellt ist. Die erfindungsgemäße Bohrhilfe 8 weist eine Durchgangsöffnung 9 und ein Anschlussteil 10 zur Verbindung mit dem Abdruck 3 auf. Das in Fig. 4 dargestellte Anschlussteil 10 der Bohrhilfe 8 ist hierfür als Negativform des Anschlussteils 7 des Abdrucks 3 und somit als Erhebung ausgebildet und weist eine in Richtung der Erhebung verlaufende Längsachse A auf.

Es ist auch möglich, dass das Anschlussteil 10 der Bohrhilfe 8 nur teilweise als Negativform des Anschlussteils 7 des Abdrucks 3 ausgebildet ist, wobei sicherzustellen ist, dass das Anschlussteil 10 der Bohrhilfe 8 bezüglich der Längsachse A winkelmäßig eindeutig zu dem Anschlussteil 7 des Abdrucks passt und dass die Bohrhilfe verwacklungsfrei mit dem Abdruck verbindbar ist. Nur durch das Verhindern eines verdrehten Einsetzens und eine verwacklungsfreie Verbindung kann gewährleistet werden, dass die Durchgangsöffnung 9 der Bohrhilfe im in den am Kiefer positionierten Abdruck eingesetzten Zustand in Richtung der im 3D-Datensatz relativ zum Kiefer festgelegten Bohrrichtung verläuft.

Die Durchgangsöffnung 9 ist als zylindrische Öffnung mit einer dem Verlauf der Öffnung folgenden Längsachse B ausgebildet und ihr Verlauf in Fig. 4 gestrichelt dargestellt. Die Durchgangsöffnung 9 ist zum Anschlussteil 10 der Bohrhilfe 8 so ausgerichtet, dass die Längsachse B der Durchgangsöffnung 9 mit der Längsachse A des Anschlussteils 10 der Bohrhilfe 8 einen Winkel α einschließt, der dem ermittelten Winkel zwischen der geplanten Bohrrichtung und dem Anschlussteil 7 des Abdrucks 3 entspricht. Des Weiteren ist die Längsachse B in Bezug auf die Längsachse A des Anschlussteils 10 der Bohrhilfe 8 winkelmäßig so ausgerichtet, wie es für die geplante Bohrrichtung in Bezug auf die Längsachse des Anschlussteils 7 des Abdrucks 3 bestimmt wurde.

Wird das Anschlussteil 10 der Bohrhilfe 8 in das Anschlussteil 7 des Abdrucks 3 eingesetzt oder auf das Anschlussteil 7 des Abdrucks 3 aufgesetzt, so dass die Längsachse A des Anschlussteils 10 der Bohrhilfe 8 mit der Längsachse A des Anschlussteils 7 des Abdrucks 3 zusammenfällt, ergibt sich ein entsprechender Winkel α zwischen der Längsachse A des Anschlussteils 7 des Abdrucks 3 und der dem Verlauf der Durchgangsöffnung 9 entsprechenden Längsachse B sowie eine winkelmäßige Ausrichtung der Durchgangsöffnung 9 bezüglich der Längsachse A des Anschlussteils 7 des Abdrucks 3, so dass die Durchgangsöffnung 9 entlang der vorher bestimmten Bohrrichtung verläuft.

Die Durchgangsöffnung 9 dient als Führung für einen Bohrer und gibt damit bei in den Abdruck 3 eingesetzter Bohrhilfe 8 eine Richtung der Bohrung relativ zum Anschlussteil 7 des Abdrucks 3 vor. Der Abdruck 3 zusammen mit der eingesetzten Bohrhilfe 8 ergibt eine erfindungsgemäße Bohrschablone 11, die in Fig. 5 dargestellt ist.

Die Durchgangsöffnung 9 der Bohrhilfe 8 kann, wie in Fig. 4 dargestellt, eine zylindrische Geometrie aufweisen, deren Durchmesser sich dahingehend nach einem Durchmesser eines zu verwendenden Bohrers für die Implantat-Bohrung richtet, dass eine sichere Führung des Bohrers während der Implantat-Bohrung möglich ist.

Die Bohrhilfe 9 kann auch einen Adapter 12 umfassen, wie er in Fig. 6 dargestellt ist. Hierzu kann die Durchgangsöffnung 9 einen zylindrischen Querschnitt aufweisen, in den ein oder mehrere Adapter 12 einsetzbar sind. Ein solcher erfindungsgemäßer Adapter 12 weist hierzu eine Außengeometrie mit einem in die Durchgangsöffnung 9 der Bohrhilfe 8 hineinpassenden längliches Ende 13 und einen dieses Ende abschließenden Teller 14 auf. In einer Ebene, die senkrecht zu einer Längsachse C des in die Durchgangsöffnung 9 der Bohrhilfe 8 hineinpassenden Endes 13 verläuft, weist der Teller 14 einen Durchmesser auf, der größer ist als der entsprechende Durchmesser des Endes 13. Hierdurch wird eine maximale Eindringtiefe des Adapters 12 beim Einbringen in die Durchgangsöffnung 9 der Bohrhilfe 8 realisiert. Weiterhin weist der Adapter 12 eine Durchgangsöffnung 15 auf, die entlang der Längsachse C verläuft und die einen Durchmesser aufweist, der dem Bohrdurchmesser einer geplanten Bohrung entspricht.

So können beispielsweise mehrere Adapter 12 mit verschiedenen Durchmessern der Durchgangsöffnung 15 vorgesehen werden, um beispielsweise eine Pilot-Bohrung mithilfe eines ersten Adapters 12 und anschließend eine Implantat-Bohrung mittels eines weiteren Adapters 12 durchzuführen.

Wird eine mindestens einen Adapter 12 umfassende Bohrhilfe 8 verwendet, so kann der Querschnitt der Durchgangsöffnung 9 der Bohrhilfe 8 eine beliebige Form haben, wobei der mindestens eine Adapter 12 ein Ende 13 mit einer entsprechenden Außengeometrie aufweist. Die Durchgangsöffnung 9 der Bohrhilfe 8 kann beispielsweise einen quadratischen Querschnitt haben und der Adapter 12 kann ein Ende 13 mit einer entsprechenden viereckigen Außengeometrie aufweisen.

In den Fig. 7 und 8 ist die Herstellung der erfindungsgemä-ßen Bohrhilfe 8 für eine Bohrschablone 11 aus einem Block 16 schematisch dargestellt. Der an einem Halter 17 gehaltene Block 16 weist entlang einer Längsachse D des Blocks 16 eine Öffnung 18 auf, die der Durchgangsöffnung 9 der herzustellenden Bohrhilfe 8 entspricht. Das Anschlussteil 10 der herzustellenden Bohrhilfe 8 wird mittels eines Schleif- oder Fräswerkzeugs 19 durch abtragende Bearbeitung an einem vom Halter 17 abgewandten Ende des Blocks hergestellt, indem die Seitenwände 10" des Anschlussteils 10 flächig und parallel zu einer Achse E ausgebildet werden, wobei die Achse E zur Längsachse D der Öffnung 18 des Blocks 16 so ausgerichtet ist, dass die geplante Bohrrichtung entlang der Längsachse A des Anschlussteils 7 des Abdrucks 3 verläuft.

Im Übergang zwischen dem Anschlussteil 10 und einem halterseitigen Ende des Blocks 16 werden des Weiteren Auflageflächen 20 ausgebildet, die zur Auflage der Bohrhilfe 8 auf der Oberseite 3' des Abdrucks 3 im Bereich um das Anschlussteil 7 herum dienen können.

Die Auflageflächen 20 können hinsichtlich der Längsachse des Anschlussteils 10 ausgebildet sein. Sie können aber auch mit der Längsachse einen Winkel einschließen. Wesentlich ist, dass sie einen definierten Tiefenstop sicherstellen. Ein Tiefenstop kann beispielsweise durch den Übergang zwischen dem Anschlussteil 10 und der Auflagefläche 20 sichergestellt werden, indem die sich am Übergang ausbildende Kante beispielsweise formschlüssig auf die sich im Übergang von den Seitenwänden 7" des Anschlussteils 7 des Abdrucks 3 und der Oberseite 3' des Abdrucks 3 ausgebildeten Schulter aufsetzbar ist.

Die in Fig. 7 dargestellte Variante sieht vor, dass das herzustellende Anschlussteil 10 der Bohrhilfe 8 mittels der Auflageflächen 20 auf der Oberseite 3 des Abdrucks 3' aufliegt, wobei das Anschlussteil 10 der Bohrhilfe zwischen den Seitenflächen 7" des Anschlussteils 7 des Abdrucks 3 geführt werden, ohne dass das Anschlussteil 10 der Bohrhilfe 8 mit dem Boden 7' des Anschlussteils 7 in Kontakt kommt. In einem solchen Fall müssen nur die parallel zur Achse E verlaufenden Seitenwände 10" und die Auflageflächen 20 aus dem Block herausgearbeitet werden.

Die in Fig. 8 dargestellte Variante sieht vor, dass das herzustellende Anschlussteil 10 der Bohrhilfe 8 beim Anbringen an dem Abdruck 3 sowohl mit der Auflagefläche 20 auf der Oberseite 3' des Abdrucks 3 als auch mit dem Boden 10' auf dem Boden 7' des Anschlussteils 7 des Abdrucks 3 in Kontakt tritt. Zusätzlich zu den Seitenwänden 10" und den Auflageflächen 20 muss in diesem Fall auch der Boden 10' des Anschlussteils durch entsprechende Bearbeitung des Blocks hergestellt werden.

Des Weiteren wäre es auch möglich das Anschlussteil 10 der Bohrhilfe 8 so auszugestalten, dass es mit dem Boden 7' und den Seitenwänden 7" des Anschlussteils 7 des Abdrucks in Kontakt tritt, ohne jedoch mit Auflageflächen 20 auf der Oberseite des Abdrucks aufzuliegen.

Ist das Anschlussteil 10 der Bohrhilfe 4 vollständig hergestellt, so wird der Blockhalter 17 und gegebenenfalls ein Teil des halterseitigen Endes des Blocks 16 entlang einer senkrecht zur Öffnung 18 bzw. zur Längsachse D des Blocks 16 verlaufenden Ebene F abgetrennt. Die Bohrhilfe 8 kann nun an dem Abdruck 3 angebracht werden, um mit diesem zusammen die erfindungsgemäße Bohrschablone 11 zu bilden.

Neben der Bohrrichtung kann mittels der erfindungsgemäßen Bohrschablone 11 auch die Bohrtiefe kontrolliert bzw. vorgegeben werden. Hierzu wird in dem 3D-Datensatz 21 neben der Bohrrichtung auch eine Bohrtiefe für die Implantat-Bohrung relativ zum Kiefer 1 festgelegt. Damit kann entsprechend der Bohrrichtung für die sich daraus ergebende Endposition des geplanten Implantat-Bohrlochs eine Position relativ zum Anschlussteil 7 des Abdrucks 3 bestimmt werden.

Diese Position kann beispielsweise relativ zu dem Boden 7' des Anschlussteils 7 des Abdrucks 3 oder relativ zu einer n die Oberseite 3' des Abdrucks 3 angrenzenden Kante bzw. Schulter des Anschlussteils 7 bestimmt werden. Dadurch kann durch die Länge der Durchgangsöffnung 9 der Bohrhilfe 8 die Eindringtiefe eines zu verwendenden Bohrers begrenzt werden. Die Länge kann vom Boden 10' des Anschlussteils 10 der Bohrhilfe 8 oder von den Auflageflächen 20 des Anschlussteils 10 der Bohrhilfe 8 oder von der sich am Übergang zwischen den Seitenwänden 10" des Anschlussteils 10 und der Auflagefläche 20 der Bohrhilfe 8 ausbildenden Kante bis zu dem der Anschlussgeometrie abgewandten Ende der Bohrhilfe gemessen bzw. festgelegt werden. Wesentlich ist, dass der Auflagepunkt bzw. die Auflagefläche der Bohrhilfe 8 mit dem Abdruck 3 bekannt ist.

Die Bohrtiefe kann demnach für einen Bohrer mit einem geeigneten Anschlag am Bohrer selbst mittels der Länge der Durchgangsöffnung 9 der Bohrhilfe durch die erfindungsgemä-ße Bohrschablone reguliert werden. Beispielsweise kann eine am Bohrer auf einer relativ zum Bohrerende festen Länge fest angebrachte Schulter als Anschlag dienen, wobei der Bohrer soweit durch die Durchgangsöffnung 9 der Bohrhilfe 8 eindringen kann, bis die Schulter zumindest teilweise auf der der Anschlussgeometrie abgewandten Seite der Bohrhilfe 8 aufliegt.

Wird ein Bohrer ohne Anschlag verwendet, so kann die Eindringtiefe des Bohrers durch Ablesen eines Wertes am verwendeten Bohrer auf Höhe des der Anschlussgeometrie abgewandten Seite der Bohrhilfe 8 aufgrund der Bekanntheit der Länge der Durchgangsöffnung 9 der Bohrhilfe 8 relativ zum Anschlussteil 7 des Abdrucks 3 bestimmt werden.

Um eine Korrelation zwischen einer Implantationsstelle und einem 3D-Datensatz mittels eines erfindungsgemäßen Abdrucks 3 mit Anschlussteil 7 herzustellen, ist es auch möglich, anstatt eines röntgenopaken und/oder in einer MRT-Aufnahme erkennbaren Hilfselements 4 ein optisches Hilfselement 22 zu verwenden und zusätzlich zu einer Röntgenaufnahme und/oder eine MRT-Aufnahme eine optische Vermessung durchzuführen.

Ein optisches Hilfelement 22, welches beispielhaft in Fig. 10 dargestellt ist, weist, wie das bereits beschriebene Hilfselement 4, ein Anschlussteil 23 auf, dessen Negativform im bzw. am Abdruck wiederzufinden ist. Das optische Hilfselement 22 ist weiterhin so ausgebildet, dass es den Abdruck 3 vollständig durchdring, d.h. dass das optische Hilfselement 22 an der Oberseite 3' in den Abdruck 3 eindringt und auch die der Oberseite 3' gegenüberliegende Unterseite 3" des Abdrucks 3 durchbricht, so dass ein Teil des optischen Hilfselements 22 über die Unterseite 3" des Abdrucks 3 hinausragt.

An dem an der Unterseite 3" austretenden Ende des optischen Hilfselements 22 ist mindestens ein Findekörper 24 angeordnet, der sich dadurch auszeichnet, dass er eine spezifische Geometrie mit eindeutigen Flächen aufweist, mittels derer die genaue Position des Findekörpers 24 in einer optischen Aufnahme ermitteln lässt. Ein Findekörper 24 kann beipielsweise als Tetraeder ausgebildet sein. Der Findekörper 24 kann so an dem Hilfselement 22 angebracht werden, dass die relative Position des Findekörpers 24 bzw. der einzelnen Flächen des Findekörpers 24 zur Anschlussgeometrie 23 des Hilfelements 22 bekannt ist.

Die Unterseite 3' des Abdrucks 3 mit dem Hilfelement 22 mit Findekörper 24 optisch vermessen und aus der optischen Aufnahme ein 3D-Datensatz 25 erzeugt, wie er beispielhaft in Fig. 11 dargestellt ist.

Dieser 3D-Datensatz 25 wird mit dem aus der Röntgenaufnahme oder der MRT-Aufnahme erzeugten 3D-Datensatz korreliert, beispielsweise anhand von Zahnoberflächen, die in beiden Messdatensätzen zu erkennen sind. In dem hierbei erzeugten Korrelationsdatensatz 25 ist die relative Position des Findekörpers 22 zur Implantationsstelle 2 ermittelbar und daraus die Position des Anschlussteils 23 des optischen Hilfselements ableitbar, wodurch wiederum die Position des Anschlussteils 7 des Abdrucks bekannt ist.

## Patentansprüche

1. Verfahren zur Korrelation einer Implantationsstelle (2) eines Kiefers (1) und eines 3D-Messdatensatzes (21) der Implantationsstelle (2) mit nachfolgenden Verfahrensschritten:
- Bereitstellen eines an der Unterseite (3") einer Negativform zumindest eines Teilbereichs eines Kiefers (1) und zumindest einer in diesem Teilbereich des Kiefers (1) liegenden Implantationsstelle (2) aufweisenden Abdrucks (3) aus einem röntgenstrahlendurchlässigen Material und/oder aus einem Material, das in einer MRT-Aufnahme möglichst wenig sichtbar ist, wobei ein Hilfselement (4) vorhanden ist, das zumindest teilweise aus einem Material besteht, das in einer Röntgenaufnahme und/oder in MR-T-Aufnahme erkennbar ist, wobei das Hilfselement (4) ein Anschlussteil (5) aufweist, das im Bereich der Negativform der Implantationsstelle (2) auf einer der Unterseite (3'') gegenüberliegenden Oberseite (3') des Abdrucks (3) am Abdruck (3) angebracht ist;
- Herstellen mindestens einer Röntgenaufnahme und/oder einer MRT-Aufnahme zumindest des Teilbereichs des Kiefers (1) und der zumindest einen in diesem Teilbereich liegenden Implantationsstelle (2), des an der Implantationsstelle (2) eingesetzten Abdrucks (3) und des mindestens einen an dem Abdruck (3) angebrachten Hilfselements (4) und Erzeugen eines 3D-Messdatensatzes (21) aus der mindestens einen Röntgenaufnahme und/oder der mindestens einen MRT-Aufnahme;
- Feststellen der Position des Anschlussteils (5) des mindestens einen Hilfselements (4) relativ zur Lage der Implantationsstelle (2) in dem 3D-Messdatensatz (21) ;
- Bereitstellung eines dem Negativ des Anschlussteils (5) des mindestens einen Hilfselements (4) entsprechenden Anschlussteils (7) an einer mit der im 3D-Messdatensatz (21) festgestellten Position des Anschlussteils (5) des Hilfselements (4) übereinstimmenden Position in dem an der Implantationsstelle (2) eingesetzten Abdruck (3) durch Entfernen des Hilfselements (4).

2. Verfahren zur Korrelation einer Implantationsstelle (2) eines Kiefers (1) und eines 3D-Messdatensatzes (21) der Implantationsstelle (2) mit nachfolgenden Verfahrensschritten:
- Bereitstellen eines an der Unterseite (3") eine Negativform zumindest eines Teilbereichs eines Kiefers (1) und zumindest einer in diesem Teilbereich des Kiefers (1) liegenden Implantationsstelle (2) aufweisenden Abdrucks (3) aus einem teilweise röntgenstrahlendurchlässigen Material und/oder einem Isolatormaterial mit mindestens einem röntgenopaken Bereich und/oder einem in MRT-Aufnahmen zu erkennenden Bereich, wobei der Abdruck (3) im Bereich der Negativform der Implantationsstelle (2) auf einer der Unterseite (3") gegenüberliegenden Oberseite (3') des Abdrucks (3) ein Anschlussteil (7) aufweist und die Lagebeziehung zwischen dem röntgenopaken Bereich und/oder dem Bereich hoher Dichte des Abdrucks (3) und dem Anschlussteil (7) des Abdrucks (3) bekannt ist;
- Herstellen mindestens einer Röntgenaufnahme und/oder einer MRT-Aufnahme zumindest des Teilbereichs des Kiefers (1) und der zumindest einen in diesem Teilbereich liegenden Implantationsstelle (2) und des an der Implantationsstelle (2) eingesetzten Abdrucks (3) und Erzeugen eines 3D-Messdatensatzes (21) aus der mindestens einen Röntgenaufnahme und/oder der mindestens einen MRT-Aufnahme;
- Feststellen der Position des röntgenopaken Bereichs des Abdrucks (3) relativ zur Implantationsstelle (2) in dem 3D-Messdatensatz (21) und bestimmen der relativen Position des Anschlussteils (7) des Abdrucks (3) zur Implantationsstelle (2) aufgrund der relativen Position des röntgenopaken Bereichs und/oder einem in MRT-Aufnahmen zu erkennenden Bereichs.

3. Verfahren zur Korrelation einer Implantationsstelle (2) eines Kiefers (1) und eines 3D-Messdatensatzes (21) der Implantationsstelle (2) mit nachfolgenden Verfahrensschritten:
- Bereitstellen eines an der Unterseite (3") einer Negativform zumindest eines Teilbereichs eines Kiefers (1) und zumindest einer in diesem Teilbereich des Kiefers (1) liegenden Implantationsstelle (2) aufweisenden Abdrucks (3) mit mindestens einem optischen Hilfselement (22), welches ein Anschlussteil (23) aufweist, das im Bereich der Negativform der Implantationsstelle (2) von einer der Unterseite (3") gegenüberliegenden Oberseite (3') des Abdrucks (3) den Abdruck (3) durchdringt und über die Unterseite (3") des Abdrucks (3) hinausreicht und an dem über die Unterseite (3") hinausragenden Ende einen fest oder abnehmbar angeordneten Findekörper (24) aufweist;
- Herstellen mindestens einer Röntgenaufnahme und/oder MRT-Aufnahme zumindest des Teilbereichs des Kiefers (1) und der zumindest einen in diesem Teilbereich liegenden Implantationsstelle (2) und Erzeugen eines 3D-Messdatensatzes (21) aus der mindestens einen Röntgenaufnahme und/oder der mindestens einen MRT-Aufnahme;
- Herstellen mindestens einer optischen Aufnahme der Unterseite des Abdrucks (3) mit eingesetztem und über die Unterseite des Abdrucks (3) hinausragendem optischen Hilfselement (22) mit Findekörper (24) und Erzeugen eines weiteren 3D-Messdatensatzes (25) aus der mindestens einen optischen Aufnahme;
- Erzeugen eines Korrelationsdatensatzes (26) durch korrelieren des 3D-Messdatensatzes (21) aus der mindestens einen Röntgenaufnahme und/oder der mindestens einen MRT-Aufnahme und des weiteren 3D-Messdatensatzes (25) aus der mindestens einen optischen Aufnahme;
- Feststellen der Position des Anschlussteils (23) des mindestens einen optischen Hilfselements (22) relativ zur Implantationsstelle (2) in dem Korrelationsdatensatz (26);
- Bereitstellung eines dem Negativ des Anschlussteils (23) des mindestens einen optischen Hilfselements (22) entsprechenden Anschlussteils (7) an einer mit der im Korrelationsdatensatz (26) festgestellten Position des Anschlussteils (23) des optischen Hilfselements (22) übereinstimmenden Position in dem an der Implantationsstelle (2) eingesetzten Abdruck (3) durch Entfernen des optischen Hilfselements (4).

4. Verfahren zur Erstellung einer Bohrschablone (11) für eine an einer Implantationsstelle (2) durchzuführende Bohrung mit nachfolgenden Verfahrensschritten:
- Bereitstellen eines an der Unterseite (3") eine Negativform zumindest eines Teilbereichs eines Kiefers (1) und zumindest einer in diesem Teilbereich des Kiefers (1) liegenden Implantationsstelle (2) aufweisenden Abdrucks (3) mit mindestens einem Anschlussteil (7) mit bekannter Anschlussgeometrie, das im Bereich der Negativform der Implantationsstelle (2) auf einer der Unterseite (3") gegenüberliegenden Oberseite (3') des Abdrucks (3) angeordnet ist und dessen Lagebeziehung zum Kiefer (1) für den am Kiefer (1) im Bereich der Implantationsstelle (2) positionierten Abdruck (3) bekannt ist;
- Festlegen einer Bohrrichtung für mindestens ein Implantat relativ zu dem Anschlussteil (7) des Abdrucks (3) anhand eines die Implantationsstelle (2) enthaltenden 3D-Datensatzes (21, 25, 26) des Kiefers (1);
- Herstellen mindestens einer Bohrhilfe (8) mit einem Anschlussteil (10) aus einem Block (16) mit einer Öffnung (18), die entlang einer Längsachse (D) des Blocks (16) durch den gesamten Block (16) verläuft, wobei das Anschlussteil (10) der Bohrhilfe (8) zumindest teilweise dem Negativ des Anschlussteils (7) des Abdrucks (3) entspricht und wobei das Anschlussteil (10) der Bohrhilfe (8) in einem Winkel zu der Öffnung (18) angeordnet wird, der einem Winkel der Bohrrichtung zu dem Anschlussteil (7) des Abdrucks (3) entspricht;
- Anbringen der mindestens einen Bohrhilfe (8) an dem Abdruck (3), wobei eine korrekte Orientierung der Bohrhilfe (8) am Abdruck (3) mittels der jeweiligen Anschlussteile (7, 10) sichergestellt wird.

5. Verfahren nach Anspruch 19, **dadurch gekennzeichnet, dass** ein Bohrdurchmesser für mindestens ein Implantat relativ zu dem Anschlussteil (7) des Abdrucks (3) festgelegt wird und ein Block (16) mit einer zylindrischen Öffnung (18) mit einem Durchmesser gemäß dem festgelegten Bohrdurchmesser verwendet wird.

6. Verfahren nach Anspruch 19, **dadurch gekennzeichnet, dass** ein Bohrdurchmesser für mindestens ein Implantat relativ zu dem Anschlussteil (7) des Abdrucks (3) festgelegt wird und ein Block (16) mit einer Öffnung (18) mit einem Durchmesser, der größer als der festgelegte Bohrdurchmesser ist, zur Herstellung der Bohrhilfe (8) verwendet wird und dass die Bohrhilfe mit einem Adapter (12) verwendet wird, der einen Bereich mit einer dem Negativ der Öffnung (18) des Blocks (16) entsprechenden Außengeometrie, einem sich diesem Bereich anschließenden Abschlussbereich (14) und einer Durchgangsöffnung (15) mit einem Innendurchmesser gemäß dem festgelegten Bohrdurchmesser aufweist und der auf der dem Anschlussteil (10) der aus dem Block (16) hergestellten Bohrhilfe (8) abgewandten Seite in die Durchgangsöffnung (9) der Bohrhilfe (8) zumindest teilweise eingesetzt wird.

7. Verfahren nach einem der Ansprüche 19 bis 21, **dadurch gekennzeichnet, dass** zumindest das Anschlussteil (10) der Bohrhilfe (8) aus dem Block (16) gefräst oder geschliffen wird.

8. Verfahren nach einem der Ansprüche 19 bis 22, **dadurch gekennzeichnet, dass** der Block (16) zumindest während der Herstellung des Anschlussteils (10) auf einem Halter (17) gehalten wird und vor dem Einfügen entlang einer senkrecht oder schräg zur Längsachse des Blocks (16) verlaufenden Abtrennfläche von dem Halter abgetrennt wird.

9. Verfahren nach einem der Ansprüche 19 bis 22, **dadurch gekennzeichnet, dass** eine Bohrtiefe für mindestens ein Implantat relativ zu dem Anschlussteil (7) des Abdrucks (3) festgelegt wird.

10. Verfahren nach einem der Ansprüche 19 bis 23, **dadurch gekennzeichnet, dass** die Länge der Öffnung (18) der aus dem Block (16) hergestellten Bohrhilfe (8) relativ zur Änschlussgeometrie bekannt ist.
